# EUROPEAN PATENT SPECIFICATION

(11) **EP 0 235 024 B1**
(45) Date of publication and mention of the grant of the patent: **30.11.1994**
(21) Application number: 87400297.5
(22) Date of filing: 10.02.1987
(51) Int. Cl.: C12M 1/40

(54) **Enzyme sensor and method of manufacture same**
Enzym-Sensor und Verfahren zur Herstellung desselben
Senseur enzymatique et sa méthode de production

(30) Priority: 10.02.1986 JP 27277/86; 08.09.1986 JP 211205/86
(43) Date of publication of application: 02.09.1987
(73) Proprietor: TERUMO KABUSHIKI KAISHA trading as TERUMO CORPORATION, Tokyo 151 (JP)
(72) Inventor: Katsube, Teruaki, Tama-shi Tokyo (JP); Araki, Tatsuo, Uruwa-shi Saitama-ken (JP); Hara, Masashi, Urawa-shi Saitama-ken (JP)
(74) Representative: Gillard, Marie-Louise

(56) References cited:
- PATENT ABSTRACTS OF JAPAN, vol. 11, no. 354 (P-638)[2801], 19 November 1987#
- TRANSDUCERS '85, INTERNATIONAL CONFERENCE ON SOLID-STATE SENSORS AND ACTUATORS, IEEE, Piscataway, NJ (US); T. MORIIZUMI et al., p. 148#
- JOURNAL OF PHYSICS E. SCIENTIFIC INSTRUMENTS, vol. 18, no. 9, September 1985, The Institute of Physics, Bristol (GB); C. NYLANDER, pp. 736-749#
- N.E.C. RESEARCH & DEVELOPMENT, no. 78, Tokyo (JP); T. KURIYAMA et al., pp. 2-3#
- BIOTECHNOLOGY & BIOENGINEERING,vol. 26, no. 6, June 1984, J. Wiley & Sons Inc., New York, NY (US); N.J. SZUMINSKY et al., pp. 642-645#
- PATENT ABSTRACTS OF JAPAN, vol. 10, no. 264 (P-495)[2320], 09 September 1986#
- CHEMICAL ABSTRACTS, vol. 98, no. 15, 1983, Columbus, OH (US); R.M. IANNIELLO et al., p. 327, no. 122322a#
- T. KATSUBE, "Drift Mechanism and Stabilization a pH Sensitive Fiels Effect Transistor", IECON'84, 22-26 October 1984, Tokio (JP); pp. 791-794#

## Description

### BACKGROUND OF THE INVENTION

### 1. Field of the Invention:

This invention relates to an enzyme sensor which uses an insulated-gate ISFET (ion-selective field-effect transistor). More particularly, the invention relates to an enzyme sensor having an enzyme-fixed membrane deposited directly on the ion-sensitive membrane of a insulated-gate ISFET employing a sapphire substrate, and to a method of manufacturing the enzyme sensor.

The term "insulated-gate ISFET" as used in the present invention refers to a FET having a structure with the ion-sensitive membrane and gate being formed in spaced relation on the same substrate.

### 2. Description of the Prior Art:

Much progress has recently been made in semiconductor technology and in IC techniques based thereon. A sensor utilizing an ISFET for measuring the concentration of hydrogen and sodium ion has been reported by P. Bergverd, T. Matsuo and K.D. Wise in I.E.E.E. Trans. Biomedical and Engineering BEM-19, 342 (1972); ibid. BEM-21, 485 (1974).

In general, an ISFET is composed of a substrate, a barrier membrane and an ion-sensitive membrane, with the ion-sensitive membrane being connected to a gate. When the ISFET is immersed in a liquid specimen, the surface potential of the ion-sensitive membrane changes in dependence upon the ion concentration. The change in potential is measured as a change in current between e.g. the source and drain, and the results are compared with the results of similar measurement taken in a standard solution, thus enabling the ion concentration of the specimen to be determined.

The above-described ISFET is converted into an enzyme sensor by fixing an enzyme on the ion-sensitive membrane thereof for the purpose of breaking down a substance to be measured and generating hydrogen ion. A conventional method used to fix an enzyme on the ion-sensitive membrane includes steps of mixing 3-aminopropyl triethoxy silane (APTE), which is one type of silane coupling agent, with distilled water, and immersing the ISFET in the solution of the mixture to effect a reaction, whereby amino groups are introduced into the ion-sensitive membrane. This is followed by coating the ion-sensitive membrane with a fixed membrane stock solution, the principle ingredient of which is glutaric dialdehyde, using a method such as dip coating. As a result, the amino and aldehyde groups form bonds of the type -CH=N-. Successive steps include dipping the above into a dilute glutaric dialdehyde solution to introduce the aldehyde groups onto the fixed membrane, then coating the result with an enzyme (e.g. urease) solution to fix the urease on the fixed membrane due a reaction between the amino groups of the urease and the aldehyde groups on the fixed membrane surface. (See "Biosensors", edited by Shuichi Suzuki, Kodansha Scientific.)

However, the enzyme sensor obtained by the foregoing method has such shortcomings as a slow speed of response and low sensitivity. The inventors have discovered that the cause of this is the enzyme-fixed membrane fixed on the ion-sensitive membrane, and has perfected the present invention on the basis of this discovery.

Another enzyme sensor conventionally available is formed about a MOSFET having a MOS substrate. This enzyme sensor also has a number of problems because of a structure in which an enzyme-fixed membrane is directly deposited on the gate region of the MOSFET. Specifically, when the sensor is immersed in a liquid specimen, the liquid seeps into the gate region. Also, the gate region is sensitive to light, as a result of which drift tends to occur. Furthermore, it is necessary to treat the material surface of the gate with a silane coupling agent in order to deposit the enzyme-fixed membrane on the gate used in this conventional enzyme sensor. As a result, the process for fabricating the sensor is complicated.

The study of insulated-gate ISFETs is progressing in order to eliminate the aforementioned drawbacks and stabilize ISFET response. With an insulated-gate ISFET, only the ion-sensitive portion need be immersed in the liquid specimen and the FET gate is structured so as to be shielded from both light and external fields, just as the source and drain portions. This is intended to stabilize response.

However, as long as an insulator is used as the ion-sensitive membrane, as in the conventional gate material, forming the insulated gate structure is difficult. In view of these circumstances, the inventors discovered previously that if an electrically conductive iridium oxide membrane is used as the ion-sensitive membrane, the iridium oxide exhibits excellent adhesion with respect to the barrier membrane, and the iridium oxide is capable of functioning as an outstanding pH sensor having good pH sensitivity (T. Katsube; 1984 Internatioal Conference on Industrial Electronics, Control and Instrumentation; October 22-26, 1984).

However, ISFETs of the type so far available generally are fabricated using a silicon substrate. When such an ISFET is used in an enzyme sensor, it is necessary to insulate the silicon substrate from the solution in which the sensor is immersed when used. To this end, an oxidized membrane is formed on the suface of the silicon substrate by providing the silicon substrate with holes and then carrying out oxidation. This approach involves a complex manufacturing process and the oxide membrane formed is brittle and tends to break. As a result, the insulating property is difficult to maintain. Furthermore, the ISFET using the silicon substrate does not possess sufficient strength, so that the enzyme sensor employing this ISFET is difficult to handle.

### SUMMARY OF THE INVENTION

An object of the present invention is to provide a novel enzyme sensor having a quick speed of response and excellent sensitivity achieved by improving the enzyme-fixed membrane.

The enzyme sensor according to the present invention is characterized by comprising an insulated-gate ISFET having an ion-sensitive membrane formed on an insulator and offset from and overlapping a gate region of said ISFET; a portion of said insulator over the gate region being cut away to leave a thin layer thereof, the ion-sensitive membrane being formed on said thin layer of the insulator and on the rest of the insulator remote from said thin layer; said ion-sensitive membrane generating an electrical potential corresponding to pH and transmitting the potential to the gate region; and an enzyme-fixed membrane having a substantially uniform thickness of from 3 to 100 µm directly coating said ion-sensitive membrane.

The ion-sensitive membrane preferably is iridium oxide or palladium oxide. The insulated-gate ISFET is preferably formed on a sapphire substrate.

The enzyme-fixed membrane contains urease as an enzyme for sensing urea concentration and contains glucose oxidase for sensing glucose concentration.

Since the enzyme sensor of the invention has the enzyme-fixed membrane provided to a thickness of 3-100 µm on the ion-sensitive membrane of the ISFET, the speed of response is high and the sensor has a satisfactory sensivity. This enables the concentration of a substance of interest in a liquid specimen to be measured quickly and reliably.

The ISFET being of the insulating gate type, only the ion-sensitive membrane portion need be immersed in the liquid specimen, it being unnecessary to immerse the FET gate, drain and source regions. Moreover, since these portions are shielded from light and external fields, response is stabilized.

Further, in accordance with the method of manufacturing the enzyme sensor of the invention, which method includes forming the ISFET and depositing the enzyme-fixed membrane on the ion-sensitive membrane of the ISFET to a thickness of 3 - 100 µm by a spin coating process, the thickness of the enzyme-fixed membrane formed on the ion-sensitive membrane can be precisely controlled. This enables the enzyme sensor to be manufactured reliably and with facility.

The inventors have performed exhaustive research in an effort to eliminate the aforementioned defects of the conventional enzyme sensor of the type having an ordinary ISFET. As a result of this research, the inventors have discovered that if the ion-sensitive membrane of an insulated-gate ISFET is treated with an aqueous solution, which ISFET includes a sapphire substrate and uses an iridium oxide or palladium oxide membrane as the ion-sensitive membrane, fixation of the enzyme can be carried out with ease and the insulated-gate ISFET having the enzyme-fixed membrane exhibits excellent sensor characteristics. The present invention has been perfected on the basis of this discovery.

Since the enzyme sensor of the invention has the above-described construction, such outstanding characteristics as the stability of the insulated-gate ISFET can be maintained. Moreover, since the sensor makes use of an enzyme in its structure, the concentration of a substrate can be measured with excellent selectivity. By virtue of the insulated gate structure, furthermore, there is a comparatively high degree of freedom in terms of designing the area, configuration and thickness of the ion-sensitive membrane. This has important practical benefits. Since the sapphire substrate has sufficient strength, the enzyme sensor of the invention can advantageously be sufficiently strong and therefore easy to handle.

It is also possible to provide a comparison FET, namely a FET which serves as a control, in which an enzyme is not fixed on the sapphire substrate, or in which a deactivated enzyme is fixed on the sapphire substrate. Accordingly, in another aspect of the invention, there is provided an enzyme sensor comprising:
- a pair of insulated-gate ISFETs formed on a substrate, each ISFET having an ion-sensitive membrane formed on an insulator and offset from and overlapping a gate region of each of said ISFETs; a portion of said insulator over the gate region being cut away to leave a thin layer thereof, the ion-sensitive membrane being formed on said thin layer of the insulator and on the rest of the insulator remote from said thin layer; said ion-sensitive membrane generating an electrical potential corresponding to pH and transmitting the potential to the gate region;
- a membrane having fixed therein an active enzyme having a substantially uniform thickness of from 3 to 100 µm directly coating said ion-sensitive membrane of one of said ISFETs to be used as an enzyme sensing electrode; and
- a membrane having fixed therein an inactivated enzyme having a substantially uniform thickness of from 3 to 100 µm directly coating said ion-sensitive membrane of another of said ISFETs to be used as a comparison electrode.

The ion-sensitive membrane preferably is iridium oxide or palladium oxide.

The pair of insulated gate ISFETs is preferably formed on a sapphire substrate.

The active enzyme may be urease for sensing urea concentration, glucose oxidase for sensing glucose concentration.

Other features and advantages of the present invention will be apparent from the following description taken in conjunction with the accompanying drawings, in which like reference characters designate the same or similar parts throughout the figures thereof.

### BRIEF DESCRIPTION OF THE DRAWINGS

Fig. 1 is a sectional view illustrating an example of the structure of an enzyme sensor according to the present invention;
Fig. 2 is a circuit diagram illustrating the circuit of a measurement apparatus associated with the enzyme sensor of the present invention;
Fig. 3 is a graph illustrating the relationship between enzyme-fixed membrane thickness and output voltage;
Fig. 4 is a graph illustrating the relationship between enzyme-fixed membrane thickness and response time;
Fig. 5 is a graph illustrating the temporal change in the output voltage of a urea sensor according to a fifth embodiment of the invention;
Fig. 6 is a graph illustrating the calibration curve of a urea sensor with respect to urea in accordance with the fifth embodiment of the invention;
Fig. 7 is a graph illustrating the temporal change in the output voltage of a glucose sensor according to a sixth embodiment of the invention;
Fig. 8 is a graph illustrating the calibration curve of a glucose sensor with respect to glucose in accordance with the sixth embodiment of the invention; and
Fig. 9 is a sectional view illustrating an example of the structure of a sapphire substrate according to the present invention.

### DESCRIPTION OF THE PREFERRED EMBODIMENTS

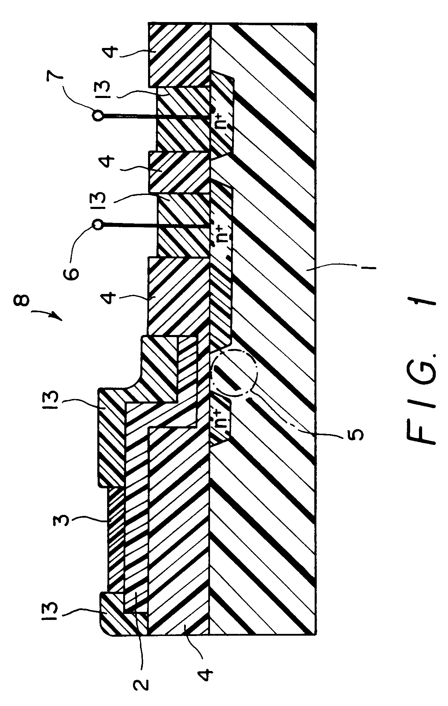

An embodiment of an enzyme sensor according to the invention and illustrated in Fig. 1 will now be described.

The enzyme sensor in this embodiment of the present invention includes a p-type silicon substrate 1 having a FET formed thereon, an insulator 4, and an ion-sensitive membrane 2 provided on the insulator 4 and having an enzyme-fixed membrane 3 at a position a short distance away from the FET. The FET has a drain 6, a source 7 and a gate 5, which is connected to the ion-sensitive membrane 2. Though the arrangement shown in Fig. 1 employs the p-type silicon substrate, the invention is not limited thereto, for a sapphire substrate may also be used. A sapphire substrate has greater strength and thus raises the strength of the enzyme sensor formed. According to the invention the insulated-gate arrangement is adopted. The reason is that with an insulated-gate ISFET, only the ion-sensitive membrane portion need be immersed in a liquid specimen, it being unnecessary to immerse the FET gate and the portions constituting the source and drain. Moreover, these portions are shielded from light and external fields, thus resulting in a more stable response.

As mentioned earlier, the term "insulated-gate ISFET" as used in the present invention refers to a FET having a structure in which the substrate, a barrier membrane and a gate are connected to a source, with the ion-sensitive membrane and gate being formed in spaced relation on the same substrate.

The FET is formed on the p- (or n-) type silicon substrate 1 by an ordinary method. For example, the FET may be formed on a p- (or n-) type silicon substrate by forming n- (or p-) type silicon in the portions to serve as the source and drain by a well-known method such as a photoresist method, discharge treatment method or thermal diffusion method or a combination thereof.

It is preferred that the position at which the FET is formed be in the vicinity of the end portion of the p-type silicon substrate. The ion-sensitive membrane 2 should be provided near the other end of the substrate remote from the gate of the FET. Such a structure enables the entirety of the p-type silicon substrate to be effectively utilized and is benificial in terms of the insulating technique.

The insulator 4 is placed on the p-type silicon substrate 1, a portion of the insulator over the gate 5 is cut away to leave a thin layer thereof, and the ion-sensitive membrane 2 is formed on this thin layer of the insulator and on the rest of the insulator remote from this portion. Silicon dioxide (SiO₂) is suitable for use as the insulator material. Examples of the material that can be used to form the ion-sensitive membrane 2 are iridium oxide (IrO₂), palladium oxide (PdO₂), silicon nitride (Si₃N₄), aluminum oxide (Al₂O₃) and tantalum oxide (Ta₂O₅). Iridium oxide and palladium oxide are especially preferred owing to their outstanding pH sensitivity.

Above all, iridium oxide is preferred as the ion-sensitive membrane formed thereby is a low-noise membrane and acts as an electrical conductor. The ion-sensitive membrane 2 is formed by depositing the abovementioned material on the insulator 4 on the p-type silicon substrate 1 by a reactive sputtering method.

The gate 5 of the FET formed on the p-type silicon substrate 1 and the ion-sensitive membrane 2 are connected via the thinned portion of the insulator 4, as set forth above. The interface potential of the ion-sensitive membrane 2 can thus be transmitted to the gate 5.

The enzyme-fixed membrane 3, which has a thickness of 3 - 100 µm, is provided on the ion-sensitive membrane 2 at a position remote from the gate 5. An enzyme-fixed membrane of such thickness exhibits excellent speed of response and a high sensitivity. The enzyme-fixed membrane 3 contains an enzyme and acts to support this enzyme on the ion-sensitive membrane 2.

The enzyme used in the present invention can be any that decomposes the particular substance of interest and produces protons. Examples are urease (for detecting urea), glucose oxidase (for detecting glucose), penicillinase (for detecting penicillin), trypsin (for detecting peptides), lipase (for detecting fatty acids) and peptidase (for detecting threonine).

A preferred method of providing the enzyme-fixed membrane 3 on the ion-sensitive membrane 2 in accordance with the invention is a lift-off method using a photoresist. An example of this method includes spin coating the ion-sensitive membrane 2 of the FET with a positive-type photoresist, exposing portions other than that at which the enzyme-fixed membrane 3 is to be formed to light to effect development, and thereafter removing the photoresist from the portion of the ion-sensitive membrane at which the enzyme-fixed membrane 3 is formed. A known photoresist can be used, such as that obtained by adding N-methyl-P-formyl stilpyridinium meto sulfate (Japanese Patent Application Laid-Open (KOKAI) No. 56-5761), polyethylene glycol methacrylate (in which e.g. benzoin ethyl ether is added as a sensitizer), polyvinyl alcohol (in which a diazido compound is added as a crosslinking agent), and polyvinyl propylene (in which a diazido compound is added as a crosslinking agent).

The portion of the ion-sensitive membrane 2 exposed (the portion on which the enzyme-fixed membrane is to be formed) and the portion of the ion-sensitive membrane 2 on which the cured photoresist has been formed are spin coated with a solution of a silane coupling solution (e.g. 1 wt-% of a 3-amino propyl triethoxy silane solution), and the result is heated (e.g. at 110°C for 5 min) to introduce the amino groups onto the surface. This is followed by immersion into a 5% glutaric aldehyde solution (e.g. for about 15 min), and then by washing and drying.

The result is then spin coated from above with an enzyme solution obtained by mixing a bovine serum albumin solution (e.g. 300 mg/ml), a urease solution (e.g. 50 mg/ml) and a glutaric aldehyde solution (e.g. 2 wt-%) with a buffer solution [e.g. PIPES NaOH buffer solution (0.1 M, pH 6.8), HEPES buffer solution (0.1 M, pH 7.5) or tris-hydrochloride solution].

The spin coating process can be carried out using a model IH-D2 spinner manufactured by Mikasa K.K. For example, carrying out spin coating at 3000 rpm for 10 min at 25°C enables the enzyme-fixed membrane 3 to be deposited on the ion-sensitive membrane 2 to a thickness of about 1 µ by a single operation.

Thus, the enzyme-fixed membrane 3 can be formed to any desired thickness. To achieve a thickness of 3 - 100 µm, the above operation using the spinner is repeated from about three to 100 times. The thickness of the enzyme-fixed membrane 3 deposited by a single operation can be changed by varying the viscosity of the enzyme solution. After the application of the enzyme solution by spin coating, the element is let stand (e.g. for not less than 30 min) and is then dipped (preferably with the application of ultrasonic waves) in a solvent (e.g. acetone) which will dissolve the cured photoresist but not the enzyme-fixed membrane, thereby removing the photoresist. The enzyme-fixed membrane 3 is thus formed on a portion of the ion-sensitive membrane 2.

Preferably, portions other than the enzyme-fixed membrane 3 and insulator 4 are provided with a protective film 13 to protect the FET.

Using the above-described method makes it possible to accurately control the thickness of the enzyme-fixed membrane and form the membrane 3 to a thickness of 3 -100 µm easily and reliably.

It is desirable to provide a comparison electrode, which serves as a control, on the p-type silicon substrate 1, the electrode having the same construction as that of the above-described enzyme sensor except for the absence of the enzyme-fixed membrane 3. Though the electrode could be provided on a separate substrate, disposing it on the same substrate is convenient in that only one sensor would need to be immersed in a liquid specimen when a measurement is taken. It is also convenient in that this will allow a fairly homogeneous FET to be formed. It is also preferred that a heat- or acid-deactivated enzyme-fixed membrane be provided on the ion-sensitive membrane of the comparison electrode. This will make it possible to ascertain the influence of drift and the like.

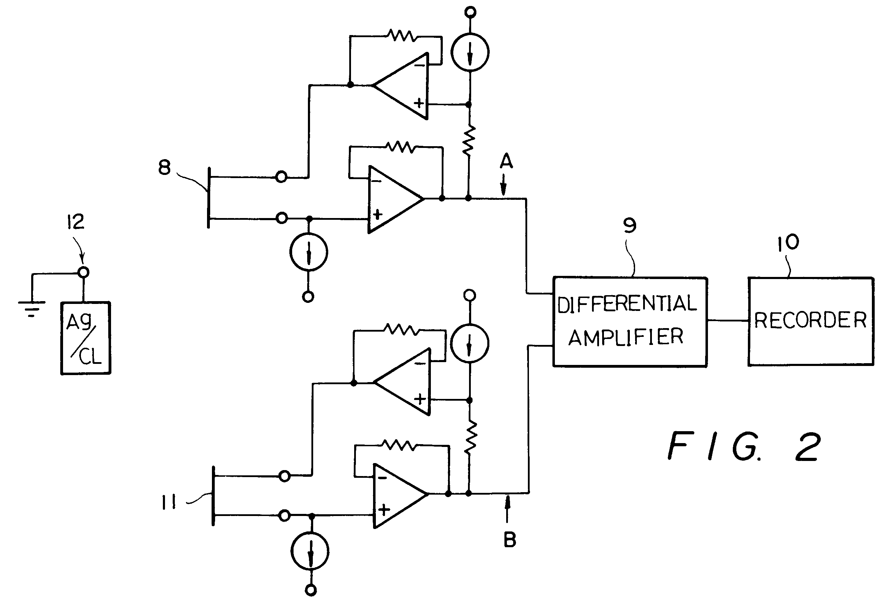

Reference will now be had to Fig. 2 to describe a measurement circuit using the enzyme sensor of the invention, which is shown at numeral 8 in Fig. 2. It should be noted that this circuit also includes a measurement circuit for the comparison electrode 11. The circuit of Fig. 2 is for directly measuring a change in the interface potential between a liquid specimen and the ion-sensitive membrane. Further, the response characteristic with respect to the liquid specimen is measured based on an differential output between the enzyme sensor 8 having the enzyme-fixed membrane and the comparison electrode 11 constituted by a FET devoid of the enzyme-fixed membrane or having a deactivated enzyme-fixed membrane.

This measurement circuit will now be described in brief.

A constant drain current flows into the enzyme sensor 8 and comparison electrode 11 due to the action of feedback circuits comprising operational amplifiers. Also, a constant voltage is impressed across the source and drain of these elements at all times. The potential of the solution is held constant by an Ag/AgCl electrode 12, and a change in the interface potential at the interface of the solution and the ion-sensitive membrane appears at terminals A, B. This change is produced by a change in the pH of the solution. These two outputs are imputted to a differential amplifier 9, which produces a differential output recorded with respect to a change in time by means of a recorder 10.

Examples of the invention will now be described.

### Examples 1 - 4

The enzyme sensor 8 using an ISFET having the structure shown in Fig. 1 was fabricated in accordance with the following method:

### (1) FET fabrication

A FET was formed in a portion of a p-type silicon substrate using a photoresist method and electrical discharge treatment in combination.

### (2) ISFET fabrication

Next, the insulator 4 (SiO₂) was provided from a position distanced from the gate portion 5 of the FET up to a portion over the gate. The insulator 4 on the gate 5 of the FET was then thinned by being cut away. An iridium oxide membrane, which eventually becomes the ion-sensitive membrane 2, was formed on the insulator 4 from a position over the gate 5 of the FET and at a position distanced from the gate 5. This membrane was formed by reactive sputtering under a vacuum of 5 x 10⁻³ Torr in pure oxygen. The membrane thickness was 800 Å. The source 7 and drain 6 of the FET were provided with respective electrodes.

### (3) Enzyme sensor fabrication

The ion-sensitive membrane 2 of the FET was then spin coated with a positive photoresist, and portions other than that (150 x 500 µm) at which the enzyme-fixed membrane 3 is to be formed were exposed to light and developed. The photoresist was then removed from the portion of the ion-sensitive membrane 2 at which the enzyme-fixed membrane 3 is to be formed.

Next, the portion of the ion-sensitive membrane 2 exposed (the portion on which the enzyme-fixed membrane is to be formed) and the portion of the ion-sensitive membrane 2 having the cured photoresist formed thereon were spin coated with a solution of a silane coupling solution (e.g. 1 wt-% of a 3-amino propyl triethoxy silane solution), and the result was heated at 110°C for 5 min to introduce the amino groups onto the surface. This was followed by dipping into a 5% glutaric aldehyde solution for about 15 min, and then by washing and drying.

The result was then spin coated from above with an enzyme solution obtained by adding 50 mg/ml of a urease solution and 2 wt-% of a glutaric aldehyde solution to 300 mg/ml of a bovine serum albumin solution (a 0.1 M HEPES buffer solution of pH 7.5) or tris-hydrochloride solution]. The spin coating process was performed for 10 min at 300 rpm under a temperature of 25°C. This procedure was repeated to fabricate a urease-fixed membrane having a thickness of 3 µm (Example 1), 5 µm (Example 2), 10 µm (Example 3) and 20 µm (Example 4). After the application of the enzyme solution by spin coating, the elements were let stand for not less than 30 min and were then dipped in acetone under the application of ultrasonic waves to remove the cured photoresist. In order to protect the FET, the protective film 13 was provided on portions other than the enzyme-fixed membrane 3 and insulator 4. Enzyme sensors were thus fabricated.

It was possible to control the thickness of the urease-fixed membrane to an accuracy of about 1 µm by using the spin coating method. The adhesion between the exposed surface of the ion-sensitive membrane 2 and the enzyme-fixed membrane membrane 3 was excellent.

### (Comparative Example 1)

A urea sensor having an urease-fixed membrane thickness of 1 µm was fabricated using the method of Example 1 with the exception of the fact that spin coating was not repeated.

### (Comparative Example 2)

A urea sensor was fabricated by coating the ion-sensitive membrane 2 of an ISFET the same as that used in Example 1 with 5 µl of a microsyringe-extracted solution obtained by dissolving urease in a solution consisting of a 0.2 M tris-hydrochloride buffer solution (pH 8.5) and 15% bovine serum albumin, blow drying the element and then adding 0.5 µl of a 25 vol-% glutaric aldehyde solution dropwise to form a urease-fixed membrane by a crosslinking reaction. The urea sensor so obtained had a urease-fixed membrane thickness of about 80 µm.

### (Experiment 1)

The output voltages and speeds of response of the urea sensors obtained in Examples 1 - 4 and Comparative Examples 1, 2 were measured by the following method:

Measurements were taken in 20 mM of a pH 7.5 HEPES buffer solution at urea concentrations of 10 mg/dl, 100 mg/dl and 1000 mg/dl. Gate bias was adjusted by an Ag/AgCl electrode, and the change in gate surface potential (output voltage) and the speed of response at such time were measured.

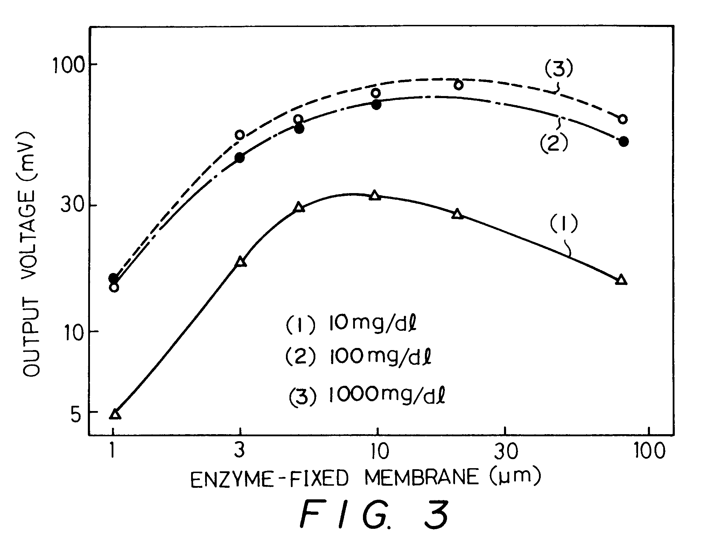

Fig. 3 illustrates the results with regard to output voltage. Output voltage is plotted along the vertical axis, and the thickness of the enzyme-fixed membrane 3 is plotted along the horizontal axis.

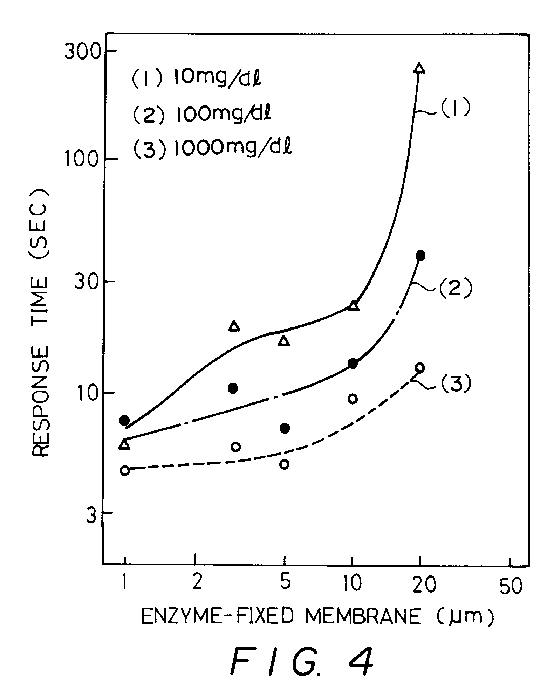

Fig. 4 illustrates the results with regard to speed of response. Response time is plotted along the vertical axis, and the thickness of the enzyme-fixed membrane 3 is plotted along the horizontal axis.

Fig. 3 shows that output voltage rises sharply with an increase in enzyme-fixed membrane thickness from 1 µm, the output voltage reaching saturation at about 10 µm and then decreasing. It was found that the sensors of Examples 1 - 4 having the enzyme-fixed membrane thicknesses of 3 - 20 µm develop satifactory output voltages and exhibit high sensitivity.

Fig. 4 shows that the speed of response rises for thinner enzyme-fixed membrane thicknesses. It was found that the speed of response is dependent upon the concentration of the substrate (urea). It was also determined that the sensors of Examples 1 - 4 having the enzyme-fixed membrane thicknesses of 3 - 20 µm exhibit a satisfactory speed of response, and that a particularly preferred enzyme-fixed membrane thickness is 3 - 10 µm.

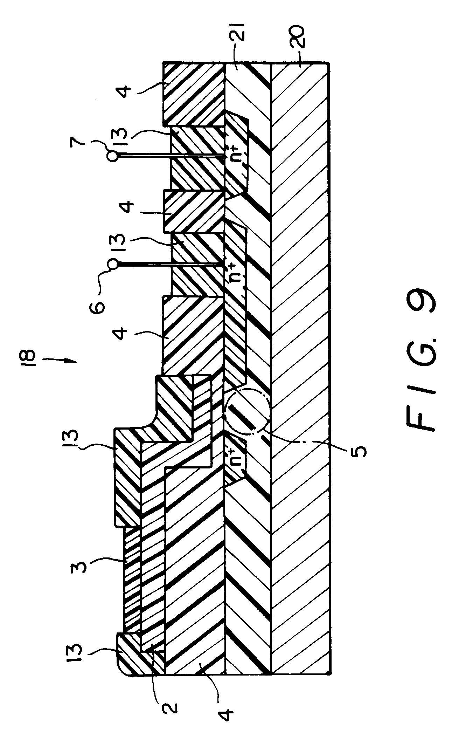

An embodiment which has the p-type silicon layer 21 in Fig. 9 places on the sapphire substrate 20 will now be described.

The enzyme sensor in this embodiment of the present invention includes a sapphire substrate 20 having a FET formed thereon, and the ion-sensitive membrane 2 provided on the insulator 4 and having the enzyme-fixed membrane 3 secured thereto at a position a short distance away from the FET. The FET has the drain 6, source 7 and gate 5, which is connected to the ion-sensitive membrane 2.

The sapphire substrate 20 preferably has a thickness of not less than 0.30 mm. Though an enzyme sensor can be formed using a thickness of less than 0.30 mm, the sensor would not have sufficient strength for use. On the other hand, a thickness of greater than 1.0 mm not only makes cutting difficult when the substrate is cut into chips but also raises the cost of material. It is preferred that a silicon layer 21 (e.g. p-type silicon) overlying the sapphire substrate 20 to form the ion-sensitive portion have a thickness of 0.53 mm ± 0.05 mm. In such case the silicon used would have a specific resistance of 20Ω·cm or less.

The FET can be formed on the sapphire substrate 20 by an ordinary method. It is generally preferred to use a FET having a silicon layer 21 provided on the sapphire substrate 20 by growing n- or p-type silicon epitaxially. If a FET having an n-type silicon layer is adopted, the FET is formed by forming p-type silicon in the portions to serve as the source and drain by a well-known method such as a photoresist method, discharge treatment method or thermal diffusion method or a combination thereof.

It is preferred that the position at which the FET is formed be in the vicinity of the end portion of the sapphire substrate. The ion-sensitive membrane 2 should be provided near the other end of the substrate remote from the gate 5 of the FET. Such a structure enables the entirety of the sapphire substrate to be effectively utilized and is benificial in terms of the insulating technique.

The ion-sensitive membrane 2 is formed near said other end of the sapphire substrate 20. If an SOS (silicon-on-sapphire) structure is used, the insulator 4 is placed thereon and the ion-sensitive membrane 2 is formed on the insulator. Silicon dioxide is suitable for use as the insulator 4. Examples of the material that can be used to form the ion-sensitive membrane 2 are iridium oxide and palladium oxide. These are preferred owing to their outstanding pH sensitivity. A suitable method of providing the ion-sensitive membrane 2 is to form reactive palladium on the sapphire substrate 20 by vapor deposition.

The gate 5 of the FET and the ion-sensitive membrane 2 are connected by an electrically conductive material (iridum oxide, palladium oxide, etc.)

The enzyme-fixed membrane 3 is secured on the ion-sensitive membrane 2, contains an enzyme and acts to support this enzyme on the ion-sensitive membrane 2.

The enzyme used in the present invention can be any that breaks down the particular substance of interest and produces protons. Examples are urease (for detecting urea), glucose oxidase (for detecting glucose), penicillinase (for detecting penicillin), trypsin (for detecting peptides), lipase [for detecting fatty acids, e.g. phosphorylase (for detecting acetylcholine)] and peptidase (for detecting threonine).

An ordinary enzyme fixing method can be used to deposit the enzyme-fixed membrane 3 on the iridium oxide or palladium oxide ion-sensitive membrane 2. An example of such a method includes steps of dissolving the enzyme in a tris-hydrochloride buffer solution to which bovine serum albumin has been added, coating the top of the ion-sensitive membrane 2 with the resulting solution and drying the same, and then adding a glutaric aldehyde solution dropwise to fix the enzyme by a crosslinking reaction. The adhesion between the enzyme-fixed membrane 3 thus obtained and an iridium oxide membrane is excellent. Though no particular limitation is imposed upon membrane thickness, the membrane usually is deposited to a thickness of 400 - 1000 Å. Preferably, the thickness of the enzyme-fixed membrane 3 is set to 50 - 100 µm.

It is desirable to provide an electrode for comparison purposes on the sapphire substrate 20, the electrode having the same construction as that of the above-described enzyme sensor except for the absence of the enzyme-fixed membrane 3. The comparison electrode is useful in observing the drift of the FET. Though the comparison electrode could be provided on a separate substrate, disposing it on the same substrate is convenient in that only one sensor would need to be immersed in a liquid specimen when a measurement is taken. It is also convenient in that this will allow a fairly homogeneous FET to be formed. It is also preferred that a heat- or acid-deactivated enzyme-fixed membrane be provided on the ion-sensitive membrane of the comparison electrode. This will make it possible to ascertain the influence of drift and the like.

The measurement circuit associated with the enzyme sensor of this embodiment of the present invention is that shown in Fig. 2, described above.

### Example 5

The enzyme sensor 18 using an insulated gate ISFET having the structure shown in Fig. 9 was fabricated in accordance with the following method:

### (1) FET fabrication

An SOS structure was formed by providing (by means of epitaxial growth) a silicon layer 21 having a width of 0.5 mm, a length of 6 mm and a thickness of 60 µm on one surface of the sapphire substrate 20 having a thickness of 350 µm. A FET was formed in a portion of the substrate on the abovementioned silicon layer 21 using a photoresist method and electrical discharge treatment in combination.

### (2) Insulated-gate ISFET fabrication

Next, the insulator 4 (SiO₂) was provided at a position distanced from the gate portion 5 of the FET on the sapphire surface having the silicon layer 21 formed thereon and was so arranged as to extend over and beyond the gate 5 of the FET. An iridium oxide membrane, which eventually becomes the ion-sensitive membrane 2, was formed on the insulator 4 by reactive sputtering under a vacuum of 5 x 10⁻³ Torr in pure oxygen. The membrane thickness was 800 Å. Thereafter, the gate 5 of the FET and the ion-sensitive membrane 2 were connected by a short conductor, and the source 7 and drain 6 were provided with respective electrodes. This was followed by insulating the FET, conductor and ion-sensitive membrane 2 with an insulator 13 (silicon nitride), leaving the electrodes completely uncovered. A portion of the insulator 13 was then removed to provide the ion-sensitive membrane 2 with an exposed surface having a surface area of 150 x 500 µm. The enzyme-fixed membrane 3 was formed on this exposed surface by the following method:

### (3) Enzyme sensor fabrication

A urea sensor was fabricated by coating the ion-sensitive membrane 2 with 5 µl of a microsyringe-extracted solution obtained by dissolving urease (manufactured by Toyo Boseki K.K.) in a solution consisting of a 0.5 M tris-hydrochloride buffer solution (pH 8.5) and 15% bovine serum albumin (manufactured by Nakarai Chemicals, Ltd.), blow drying the element and then adding 0.5 µl of a 25 vol-% glutaric aldehyde solution dropwise to form a urease-fixed membrane by a crosslinking reaction.

The adhesion between the urease enzyme-fixed membrane 3 thus obtained and the iridium oxide membrane was excellent The results of a durability test in which measurements were repeated over a period of three weeks demonstrated that there was no deterioration and no decline in sensor characteristics.

### Example 6

A glucose sensor was fabricated in the same way as that having the above-described urease-fixed membrane with the exception of the fact that glucose oxidase (manufactured by Nagase Seikagaku Kogyo K.K.) was substituted for the urease. The adhesion between the deposited glucose oxidase enzyme-fixed membrane 3 and the iridium oxide membrane was excellent, just as in Example 5.

The following experiments were conducted using the enzyme sensor fabricated as set forth above:

### (Experiment 2)

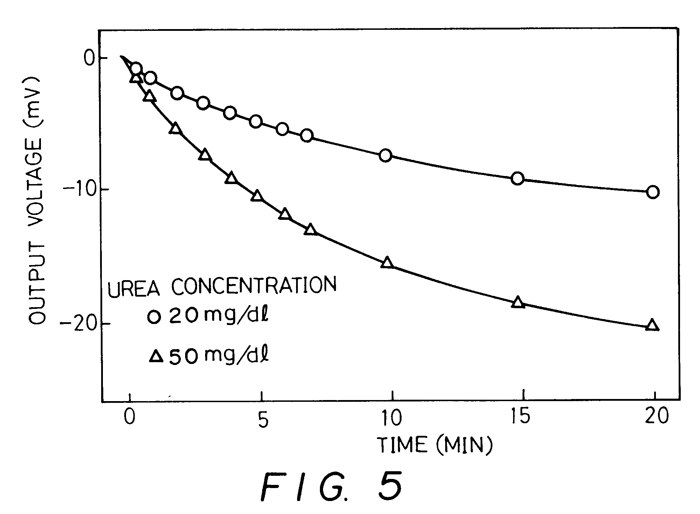

The output response characteristic of the urea sensor fabricated in accordance with Example 5 was investigated in the following manner: The change in output voltage with time when urea was added to 0.01 M of a tris-hydrochloride buffer solution (pH 7.0) so as to give urea concentrations of 20 mg/dl and 50 mg/dl was investigated. Potential was measured with respect to an Ag/AgCl electrode at a temperature of 30°C. The results are shown in Fig. 5.

A large though gentle change in output voltage was observed, as indicated by Fig. 5. The speed of response was on the same order as that of an ordinary ISFET.

### (Experiment 3)

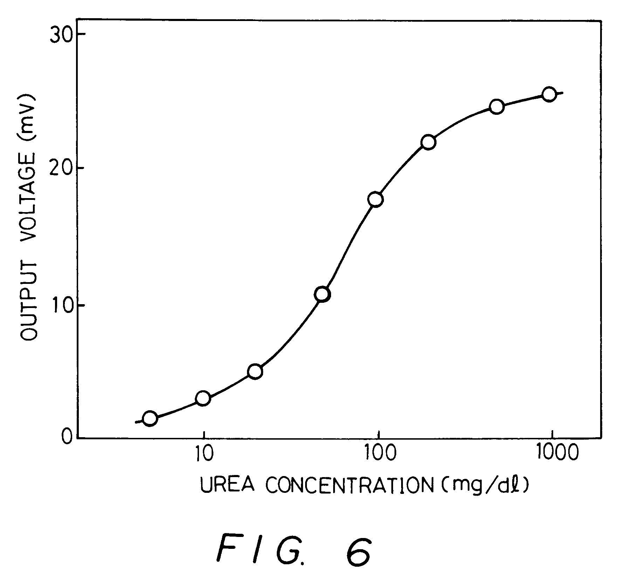

A calibration curve with regard to urea was prepared for the urea sensor fabricated in accordance with Example 5. This experiment was conducted in the same manner as Experiment 2 except for the fact that the urea concentration was varied over the range 5 - 1000 mg/dl. Fig. 6 illustrates a calibration curve prepared on the basis of output voltage after the elapse of 5 min following the addition of urea.

The output voltage exhibited a change of about 15 mV within a urea concentration range of 10 - 100 mg/dl, as Fig. 6 indicates. It was found from this fact that the urea sensor of the invention is suitable for practical use.

### (Experiment 4)

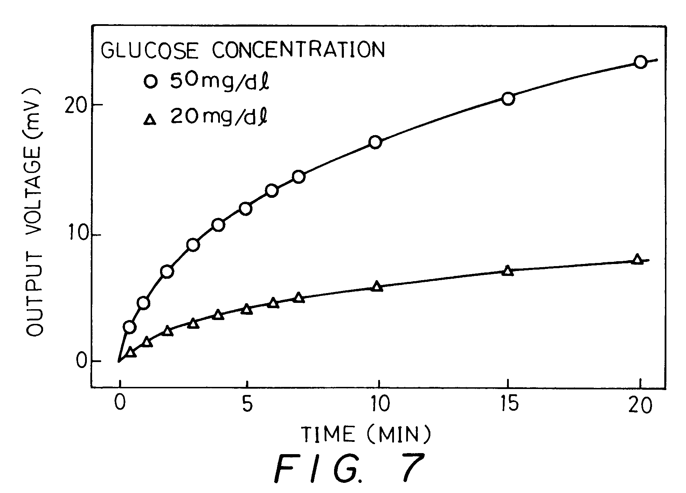

The output response characteristic of the glucose sensor fabricated in accordance with Example 6 was investigated as in Example 2. The results are shown in Fig. 7.

### (Experiment 5)

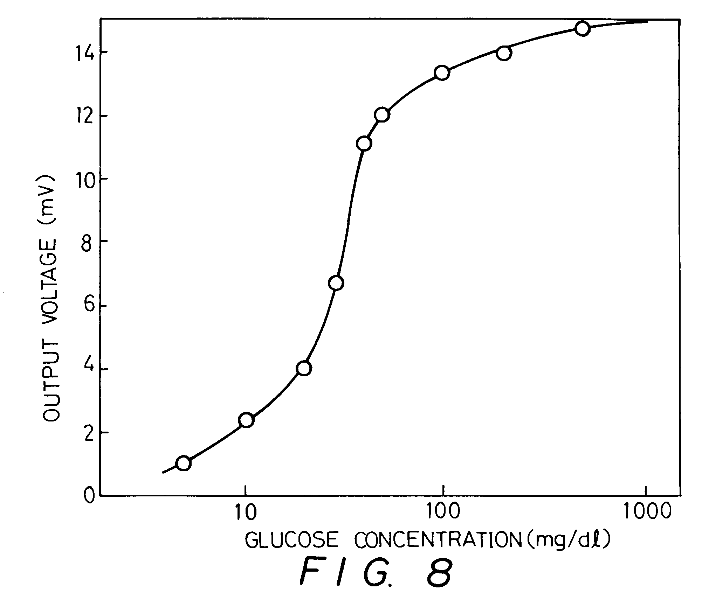

A calibration curve with regard to glucose was prepared for the glucose sensor fabricated in accordance with Example 5. Fig. 8 shows the results. The output voltage exhibited a change of about 11 mV within a urea concentration range of 10 - 100 mg/dl, as Fig. 8 indicates. It was found from this fact that the glucose sensor of the invention is suitable for practical use, just as the urea sensor mentioned above.

## Claims

1. An enzyme sensor comprising:
- an insulated-gate ISFET having an ion-sensitive membrane (2) formed on an insulator (4) and offset from and overlapping a gate region (5) of said ISFET; a portion of said insulator (4) over the gate region (5) being cut away to leave a thin layer thereof, the ion-sensitive membrane (2) being formed on said thin layer of the insulator (4) and on the rest of the insulator (4) remote from said thin layer; said ion-sensitive membrane (2) generating an electrical potential corresponding to pH and transmitting the potential to the gate region (5); and
- an enzyme-fixed membrane (3) having a substantially uniform thickness of from 3 to 100 µm directly coating said ion-sensitive membrane (2).

2. The enzyme sensor according to claim 1, wherein said insulated-gate ISFET is formed on a sapphire substrate (20).

3. The enzyme sensor according to claim 1 or 2, wherein said ion-sensitive membrane (2) is iridium oxide.

4. The enzyme sensor according to claim 1 or 2, wherein said ion-sensitive membrane (2) is palladium oxide.

5. The enzyme sensor according to any one of claims 1 to 4, wherein said enzyme-fixed membrane (3) contains urease as an enzyme for sensing urea concentration.

6. The enzyme sensor according to any one of claims 1 to 4, wherein said enzyme-fixed membrane (3) contains glucose oxydase as an enzyme for sensing glucose concentration.

7. An enzyme sensor comprising:
- a pair of insulated-gate ISFETs formed on a substrate, each ISFET having an ion-sensitive membrane formed on an insulator and offset from and overlapping a gate region of each of said ISFETs; a portion of said insulator over the gate region being cut away to leave a thin layer thereof, the ion-sensitive membrane being formed on said thin layer of the insulator and on the rest of the insulator remote from said thin layer; said ion-sensitive membrane generating an electrical potential corresponding to pH and transmitting the potential to the gate region;
- a membrane having fixed therein an active enzyme having a substantially uniform thickness of from 3 to 100 µm directly coating said ion-sensitive membrane of one of said ISFETs to be used as an enzyme sensing electrode (8); and
- a membrane having fixed therein an inactivated enzyme having a substantially uniform thickness of from 3 to 100 µm directly coating said ion-sensitive membrane of another of said ISFETs to be used as a comparison electrode (11).

8. The enzyme sensor according to claim 7, wherein said pair of insulated gate ISFETs is formed on a sapphire substrate.

9. The enzyme sensor according to claim 7 or 8, wherein said ion-sensitive membrane is iridium oxide.

10. The enzyme sensor according to claim 7 or 8, wherein said ion-sensitive membrane is palladium oxide.

11. The enzyme sensor according to any one of claims 7 to 10, wherein said enzyme is urease for sensing urea concentration.

12. The enzyme sensor according to any one of claims 7 to 10, wherein said enzyme is glucose oxidase for sensing glucose concentration.

## Patentansprüche

1. Enzymsensor, der umfaßt:
- einen ISFET mit isoliertem Gate, der eine ionenempfindliche Membran (2) aufweist, die auf einem Isolator (4) und abgesetzt von einem Gate-Bereich (5) des ISFET und diesen überlappend erzeugt ist; wobei über dem Gate-Bereich (5) ein Teil des Isolators (4) abgetragen ist und eine dünne Schicht davon zurückbleibt, die ionenempfindliche Membran (2) auf der dünnen Schicht des Isolators (4) und auf dem Rest des Isolators (4) - entfernt von der dünnen Schicht - erzeugt ist; die ionenempfindliche Membran (2) ein elektrisches Potential entsprechend dem pH erzeugt und das Potential zum Gate-Bereich (5) leitet; und
- eine Membran (3) mit fixiertem Enzym, die eine im wesentlichen einheitliche Dicke von 3 bis 100 µm aufweist und die ionenempfindliche Membran (2) direkt beschichtet.

2. Enzymsensor nach Anspruch 1, wobei der ISFET mit isoliertem Gate auf einem Saphirsubstrat (20) erzeugt ist.

3. Enzymsensor nach Anspruch 1 oder 2, wobei die ionenempfindliche Membran (2) aus Iridiumoxid besteht.

4. Enzymsensor nach Anspruch 1 oder 2, wobei die ionenempfindliche Membran (2) aus Palladiumoxid besteht.

5. Enzymsensor nach irgendeinem der Ansprüche 1 bis 4, wobei die Membran (3) mit dem fixierten Enzym Urease als Enzym zur Messung der Harnstoffkonzentration enthält.

6. Enzymsensor nach irgendeinem der Ansprüche 1 bis 4, wobei die Membran (3) mit dem fixierten Enzym Glucoseoxidase als Enzym zur Messung der Glucosekonzentration enthält.

7. Enzymsensor, der umfaßt:
- ein Paar von ISFETs mit isoliertem Gate, die auf einem Substrat erzeugt sind, wobei jeder ISFET eine ionenempfindliche Membran aufweist, die auf einem Isolator und abgesetzt von einem Gate-Bereich jedes der ISFETs und diesen überlappend erzeugt ist; wobei über dem Gate-Bereich ein Teil des Isolators abgetragen ist und eine dünne Schicht davon zurückbleibt, die ionenempfindliche Membran auf der dünnen Schicht des Isolators und auf dem Rest des Isolators - entfernt von der dünnen Schicht - erzeugt ist; die ionenempfindliche Membran ein elektrisches Potential entsprechend dem pH erzeugt und das Potential zum Gate-Bereich leitet; und
- eine Membran mit einem darin fixierten aktiven Enzym, die eine im wesentlichen einheitliche Dicke von 3 bis 100 µm aufweist und die ionenempfindliche Membran eines der ISFETs, der als Enzymsensorelektrode (8) dienen soll, direkt beschichtet; und
- eine Membran mit einem darin fixierten inaktiven Enzym, die eine im wesentlichen einheitliche Dicke von 3 bis 100 µm aufweist und die ionenempfindliche Membran eines anderen der ISFETs, der als Vergleichselektrode (11) dienen soll, direkt beschichtet.

8. Enzymsensor nach Anspruch 7, wobei das Paar der ISFETs mit isoliertem Gate auf einem Saphirsubstrat erzeugt ist.

9. Enzymsensor nach Anspruch 7 oder 8, wobei die ionenempfindliche Membran aus Iridiumoxid besteht.

10. Enzymsensor nach Anspruch 7 oder 8, wobei die ionenempfindliche Membran aus Palladiumoxid besteht.

11. Enzymsensor nach irgendeinem der Ansprüche 7 bis 10, wobei das Enzym zur Messung der Harnstoffkonzentration Urease ist.

12. Enzymsensor nach irgendeinem der Ansprüche 7 bis 10, wobei das Enzym zur Messung der Glucosekonzentration Glucoseoxidase ist.

## Revendications

1. Détecteur enzymatique comprenant :
- un ISFET à grille isolée comportant une membrane sensible aux ions (2) formée sur un isolant (4) et décalée par rapport à une région grille (5) dudit ISFET et la surplombant ; une partie dudit isolant (4) se trouvant au-dessus de la région grille (5) étant découpée pour laisser une couche mince, la membrane sensible aux ions (2) étant formée sur ladite couche mince de l'isolant (4) et sur le reste de l'isolant (4) éloigné de ladite couche mince ; ladite membrane sensible aux ions (2) générant un potentiel électrique correspondant au pH et transmettant le potentiel à la région grille (5) et
- une membrane à enzyme fixée (3), ayant une épaisseur sensiblement uniforme comprise entre 3 et 100 µm, appliquée directement sur ladite membrane sensible aux ions (2).

2. Détecteur enzymatique selon la revendication 1, dans lequel ledit ISFET à grille isolée est formé sur un substrat en saphir (20).

3. Détecteur enzymatique selon la revendication 1 ou 2, dans lequel ladite membrane sensible aux ions (2) est de l'oxyde d'iridium.

4. Détecteur enzymatique selon la revendication 1 ou 2, dans lequel ladite membrane sensible aux ions (2) est de l'oxyde de palladium.

5. Détecteur enzymatique selon l'une quelconque des revendications 1 à 4, dans lequel ladite membrane à enzyme fixée (3) contient de l'uréase comme enzyme destinée à détecter la concentration durée.

6. Détecteur enzymatique selon l'une quelconque des revendications 1 à 4, dans lequel ladite membrane à enzyme fixée (3) contient de la glucose oxydase comme enzyme destinée à détecter la concentration de glucose.

7. Détecteur enzymatique comprenant :
- une paire de ISFET à grille isolée formés sur un substrat, chaque ISFET comportant une membrane sensible aux ions formée sur un isolant et décalée par rapport à une région grille de chacun des ISFET et la surplombant ; une partie dudit isolant se trouvant au-dessus de la région grille étant découpée pour laisser une couche mince, la membrane sensible aux ions étant formée sur ladite couche mince de l'isolant et sur le reste de l'isolant éloigné de ladite couche mince ; ladite membrane sensible aux ions générant un potentiel électrique correspondant au pH et transmettant le potentiel à la région grille ;
- une membrane sur laquelle est fixée une enzyme active, ayant une épaisseur sensiblement uniforme comprise entre 3 et 100 µm, étant appliquée directement sur ladite membrane sensible aux ions de l'un desdits ISFET pour être utilisée comme électrode de détection enzymatique (8); et
- une membrane sur laquelle est fixée une enzyme inactivée, ayant une épaisseur sensiblement uniforme comprise entre 3 et 100 µm, étant appliquée directement sur ladite membrane sensible aux ions d'un autre desdits ISFET pour être utilisée comme électrode de comparaison (11).

8. Détecteur enzymatique selon la revendication 7, dans lequel ladite paire d'ISFET à grille isolée est formée sur un substrat en saphir.

9. Détecteur enzymatique selon la revendication 7 ou 8, dans lequel ladite membrane sensible aux ions est de l'oxyde d'iridium.

10. Détecteur enzymatique selon la revendication 7 ou 8, dans lequel ladite membrane sensible aux ions est de l'oxyde de palladium.

11. Détecteur enzymatique selon l'une quelconque des revendications 7 à 10, dans lequel ladite enzyme est de l'uréase destinée à détecter la concentration d'urée.

12. Détecteur enzymatique selon l'une quelconque des revendications 7 à 10, dans lequel ladite enzyme est de la glucose oxydase destinée à détecter la concentration de glucose.
